# EUROPEAN PATENT APPLICATION

(11) **EP 4 840 864 A1**
(43) Date of publication of application: **18.11.2026**
(21) Application number: 25738507.0
(22) Date of filing: 09.01.2025
(51) Int. Cl.: C07D 519/00, A61K 31/519, A61P 35/00

(54) **CRYSTAL FORM OF PYRIDOPYRIMIDINE DERIVATIVE, PREPARATION METHOD THEREFOR AND USE THEREOF**

(30) Priority: 10.01.2024 CN 202410040440; 06.01.2025 CN 202510018660
(71) Applicant: Medshine Discovery Inc., Nanjing, Jiangsu 210032 (CN)
(72) Inventor: FU, Zhifei, Shanghai 200131 (CN); LUO, Miaorong, Shanghai 200131 (CN); ZHANG, Yang, Shanghai 200131 (CN)
(74) Representative: Dai, Simin
(86) International application number: PCT/CN2025/071520
(87) International publication number: WO 2025/148979

(57) **Abstract**

Provided are a crystal form of a pyridopyrimidine derivative, a preparation method therefor, a pharmaceutical composition thereof and the use thereof. Specifically disclosed are a crystal form of a compound of formula (I), a preparation method therefor, a pharmaceutical composition thereof and the use thereof.

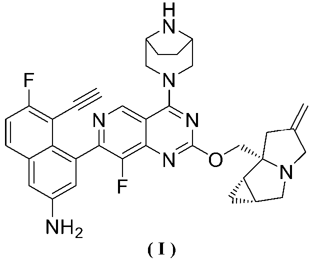

## Description

### CROSS-REFERENCE TO RELATED APPLICATIONS

The present disclosure claims the benefit and priority of Chinese Patent Application No. CN202410040440.3, filed with the China National Intellectual Property Administration on January 10, 2024, and Chinese Patent Application No. CN202510018660.0, filed with the China National Intellectual Property Administration on January 06, 2025, the entire contents of which are incorporated herein by reference.

### TECHNICAL FIELD

The present disclosure provides a crystal form of a pyridopyrimidine derivative, a preparation method therefor, and a use thereof, and specifically discloses a crystal form of a compound of formula (I), a preparation method therefor, and a use thereof.

### BACKGROUND

Mutations in NRAS, HRAS, and KRAS within the RAS family account for nearly one-quarter of all human cancers, making them one of the most common cancer-related gene mutations. These mutations cover almost all cancer types and cause 1 million deaths globally each year. Among them, KRAS is the most common oncogene (accounting for 85% of all RAS mutations) and is present in 90% of pancreatic cancers, 30-40% of colon cancers, and 15-20% of lung cancers (mostly non-small cell lung cancer). Depending on the specific mutations present, G12C, G12D, and G12R are the most common KRAS mutations in patients. In addition to these, there are G12A, G12S, G12V, and the like.

The KRAS protein belongs to the small GTPase family, possesses GTPase hydrolytic activity, and participates in various cell signaling pathways related to cell survival and growth. KRAS is in an activated state when bound to GTP (guanosine triphosphate), which can activate a series of downstream pathways. After the bound GTP is hydrolyzed into GDP (guanosine diphosphate), KRAS enters an inactivated state. Under normal conditions, once KRAS is activated, it is immediately inactivated under the action of its own hydrolase. However, oncogenic mutated KRAS exhibits impaired GTPase activity, making it more prone to remaining in the GTP-bound state. This leads to continuous activation of the KRAS protein, which in turn continuously activates downstream pathways such as the RAF/MEK/ERK and PI3K/Akt pathways, causing continuous cell proliferation, differentiation, tumor formation, and promoting tumor progression. KRAS G12D mutations account for 20%-50% of all KRAS-mutated tumors; however, no KRAS G12D drug has been marketed to date. Therefore, researching small-molecule oral inhibitors capable of inhibiting KRAS G12D is of extremely high value for the treatment of malignant tumors.

### SUMMARY

The present disclosure provides a crystal form A of a compound of formula (I), wherein the crystal form A has an X-ray powder diffraction pattern using Cu Kα radiation comprising characteristic diffraction peaks at the following 2θ angles: 8.09°±0.2°, 21.34°±0.2°, 24.74°±0.2°, and 25.26°±0.2°,

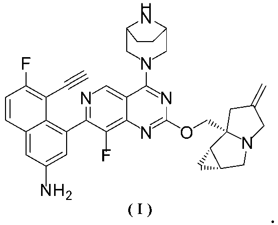

In some embodiments of the present disclosure, the X-ray powder diffraction pattern using Cu Kα radiation of the above-mentioned crystal form A comprises characteristic diffraction peaks at the following 2θ angles: 8.09°±0.2°, 19.18°±0.2°, 19.49°±0.2°, 21.34°±0.2°, 24.74°±0.2°, and 25.26°±0.2°.

In some embodiments of the present disclosure, the X-ray powder diffraction pattern using Cu Kα radiation of the above-mentioned crystal form A comprises characteristic diffraction peaks at the following 2θ angles: 8.09°±0.15°, 19.18°±0.15°, 19.49°±0.15°, 21.34°±0.15°, 24.74°±0.15°, and 25.26°±0.15°.

In some embodiments of the present disclosure, the X-ray powder diffraction pattern using Cu Kα radiation of the above-mentioned crystal form A comprises characteristic diffraction peaks at the following 2θ angles: 8.09°±0.2°, 9.46°±0.2°, 13.95°±0.2°, 19.18°±0.2°, 19.49°±0.2°, 21.34°±0.2°, 24.74°±0.2°, and 25.26°±0.2°.

In some embodiments of the present disclosure, the X-ray powder diffraction pattern using Cu Kα radiation of the above-mentioned crystal form A comprises characteristic diffraction peaks at the following 2θ angles: 8.09°±0.15°, 9.46°±0.15°, 13.95°±0.15°, 19.18°±0.15°, 19.49°±0.15°, 21.34°±0.15°, 24.74°±0.15°, and 25.26°±0.15°.

In some embodiments of the present disclosure, the X-ray powder diffraction pattern using Cu Kα radiation of the above-mentioned crystal form A comprises characteristic diffraction peaks at the following 2θ angles: 8.09°±0.2°, 9.46°±0.2°, 13.95°±0.2°, 15.28°±0.2°, 17.12°±0.2°, 19.18°±0.2°, 19.49°±0.2°, 21.34°±0.2°, 24.74°±0.2°, and 25.26°±0.2°.

In some embodiments of the present disclosure, the X-ray powder diffraction pattern using Cu Kα radiation of the above-mentioned crystal form A comprises characteristic diffraction peaks at the following 2θ angles: 8.09°±0.15°, 9.46°±0.15°, 13.95°±0.15°, 15.28°±0.15°, 17.12°±0.15°, 19.18°±0.15°, 19.49°±0.15°, 21.34°±0.15°, 24.74°±0.15°, and 25.26°±0.15°.

In some embodiments of the present disclosure, the X-ray powder diffraction pattern using Cu Kα radiation of the above-mentioned crystal form A comprises characteristic diffraction peaks at the following 2θ angles: 8.09°±0.2°, 9.46°±0.2°, 13.95°±0.2°, 15.28°±0.2°, 17.12°±0.2°, 19.18°±0.2°, 19.49°±0.2°, 20.82°±0.2°, 21.34°±0.2°, 24.74°±0.2°, 25.26°±0.2°, and 28.32°±0.2°.

In some embodiments of the present disclosure, the X-ray powder diffraction pattern using Cu Kα radiation of the above-mentioned crystal form A comprises characteristic diffraction peaks at the following 2θ angles: 8.09°±0.15°, 9.46°±0.15°, 13.95°±0.15°, 15.28°±0.15°, 17.12°±0.15°, 19.18°±0.15°, 19.49°±0.15°, 20.82°±0.15°, 21.34°±0.15°, 24.74°±0.15°, 25.26°±0.15°, and 28.32°±0.15°.

In some embodiments of the present disclosure, the X-ray powder diffraction pattern using Cu Kα radiation of the above-mentioned crystal form A comprises characteristic diffraction peaks at the following 2θ angles: 8.09°±0.2°, 9.46°±0.2°, 12.10°±0.2°, 13.95°±0.2°, 15.28°±0.2°, 16.39°±0.2°, 17.12°±0.2°, 19.18°±0.2°, 19.49°±0.2°, 20.82°±0.2°, 21.34°±0.2°, 22.22°±0.2°, 24.74°±0.2°, 25.26°±0.2°, 27.99°±0.2°, and 28.32°±0.2°.

In some embodiments of the present disclosure, the X-ray powder diffraction pattern using Cu Kα radiation of the above-mentioned crystal form A comprises characteristic diffraction peaks at the following 2θ angles: 8.09°±0.15°, 9.46°±0.15°, 12.10°±0.15°, 13.95°±0.15°, 15.28°±0.15°, 16.39°±0.15°, 17.12°±0.15°, 19.18°±0.15°, 19.49°±0.15°, 20.82°±0.15°, 21.34°±0.15°, 22.22°±0.15°, 24.74°±0.15°, 25.26°±0.15°, 27.99°±0.15°, and 28.32°±0.15°.

In some embodiments of the present disclosure, the X-ray powder diffraction pattern using Cu Kα radiation of the above-mentioned crystal form A comprises characteristic diffraction peaks at the following 2θ angles: 8.09°±0.2°, 9.46°±0.2°, 12.10°±0.2°, 12.78°±0.2°, 13.06°±0.2°, 13.95°±0.2°, 15.28°±0.2°, 16.39°±0.2°, 17.12°±0.2°, 18.56°±0.2°, 18.86°±0.2°, 19.18°±0.2°, 19.49°±0.2°, 19.84°±0.2°, 20.82°±0.2°, 21.34°±0.2°, 22.22±0.2°, 22.96°±0.2°, 23.58°±0.2°, 24.11°±0.2°, 24.74°±0.2°, 25.26°±0.2°, 27.99°±0.2°, 28.32°±0.2°, 29.00°±0.2°, and 30.21°±0.2°.

In some embodiments of the present disclosure, the X-ray powder diffraction pattern using Cu Kα radiation of the above-mentioned crystal form A comprises characteristic diffraction peaks at the following 2θ angles: 7.29°±0.2°, 8.09°±0.2°, 9.46°±0.2°, 9.75°±0.2°, 11.06°±0.2°, 12.10°±0.2°, 12.78°±0.2°, 13.06°±0.2°, 13.95°±0.2°, 14.87°±0.2°, 15.28°±0.2°, 16.39°±0.2°, 17.12°±0.2°, 17.54°±0.2°, 18.56°±0.2°, 18.86°±0.2°, 19.18°±0.2°, 19.49°±0.2°, 19.84°±0.2°, 20.82°±0.2°, 21.34°±0.2°, 22.22°±0.2°, 22.96°±0.2°, 23.58°±0.2°, 24.11°±0.2°, 24.74°±0.2°, 25.26°±0.2°, 25.73°±0.2°, 26.11°±0.2°, 26.50°±0.2°, 26.64°±0.2°, 27.54°±0.2°, 27.99°±0.2°, 28.32°±0.2°, 28.78°±0.2°, 29.00°±0.2°, 30.21°±0.2°, 30.50°±0.2°, 31.03°±0.2°, 31.99°±0.2°, 33.33°±0.2°, 33.69°±0.2°, 34.01°±0.2°, 34.39°±0.2°, 34.81°±0.2°, 35.48°±0.2°, 35.82°±0.2°, 36.37°±0.2°, 36.63°±0.2°, 37.34°±0.2°, 37.78°±0.2°, 38.39°±0.2°, 38.71°±0.2°, 39.14°±0.2°, and 39.57°±0.2°

In some embodiments of the present disclosure, the X-ray powder diffraction pattern using Cu Kα radiation of the above-mentioned crystal form A comprises characteristic diffraction peaks at the following 2θ angles: 8.09°±0.2°, 21.34°±0.2°, 24.74°±0.2°, 25.26°±0.2°, and/or 7.29°±0.2°, and/or 9.46°±0.2°, and/or 9.75°±0.2°, and/or 11.06°±0.2°, and/or 12.10°±0.2°, and/or 12.78°±0.2°, and/or 13.06°±0.2°, and/or 13.95°±0.2°, and/or 14.87°±0.2°, and/or 15.28°±0.2°, and/or 16.39°±0.2°, and/or 17.12°±0.2°, and/or 17.54°±0.2°, and/or 18.56°±0.2°, and/or 18.86°±0.2°, and/or 19.18°±0.2°, and/or 19.49°±0.2°, and/or 19.84°±0.2°, and/or 20.82°±0.2°, and/or 22.22°±0.2°, and/or 22.96°±0.2°, and/or 23.58°±0.2°, and/or 24.11°±0.2°, and/or 25.73°±0.2°, and/or 26.11°±0.2°, and/or 26.50°±0.2°, and/or 26.64°±0.2°, and/or 27.54°±0.2°, and/or 27.99°±0.2°, and/or 28.32°±0.2°, and/or 28.78°±0.2°, and/or 29.00°±0.2°, and/or 30.21°±0.2°, and/or 30.50°±0.2°, and/or 31.03°±0.2°, and/or 31.99°±0.2°, and/or 33.33°±0.2°, and/or 33.69°±0.2°, and/or 34.01°±0.2°, and/or 34.39°±0.2°, and/or 34.81°±0.2°, and/or 35.48°±0.2°, and/or 35.82°±0.2°, and/or 36.37°±0.2°, and/or 36.63°±0.2°, and/or 37.34°±0.2°, and/or 37.78°±0.2°, and/or 38.39°±0.2°, and/or 38.71°±0.2°, and/or 39.14°±0.2°, and/or 39.57°±0.2°.

In some embodiments of the present disclosure, the X-ray powder diffraction pattern using Cu Kα radiation of the above-mentioned crystal form A comprises characteristic diffraction peaks at the following 2θ angles: 8.09°, 9.46°, 12.10°, 13.95°, 15.28°, 16.39°, 17.12°, 19.18°, 19.49°, 20.82°, 21.34°, 22.22°, 24.74°, 25.26°, 27.99°, and 28.32°.

In some embodiments of the present disclosure, the X-ray powder diffraction pattern using Cu Kα radiation of the above-mentioned crystal form A comprises characteristic diffraction peaks at the following 2θ angles: 7.29°, 8.09°, 9.46°, 9.75°, 11.06°, 12.10°, 12.78°, 13.06°, 13.95°, 14.87°, 15.28°, 16.39°, 17.12°, 17.54°, 18.56°, 18.86°, 19.18°, 19.49°, 19.84°, 20.82°, 21.34°, 22.22°, 22.96°, 23.58°, 24.11°, 24.74°, 25.26°, 25.73°, 26.11°, 26.50°, 26.64°, 27.54°, 27.99°, 28.32°, 28.78°, 29.00°, 30.21°, 30.50°, 31.03°, 31.99°, 33.33°, 33.69°, 34.01°, 34.39°, 34.81°, 35.48°, 35.82°, 36.37°, 36.63°, 37.34°, 37.78°, 38.39°, 38.71°, 39.14°, and 39.57°.

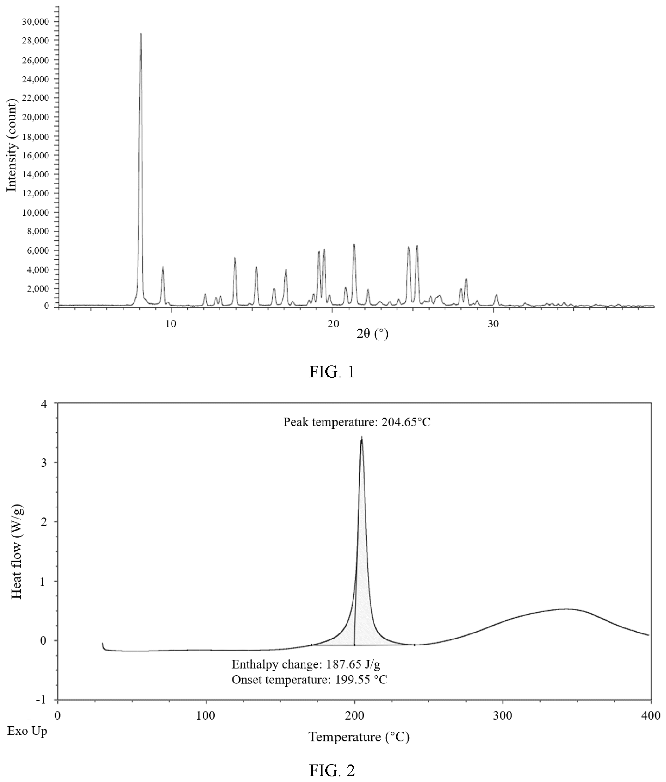

In some embodiments of the present disclosure, the above-mentioned crystal form A has an XRPD pattern substantially as shown in FIG. 1.

In some embodiments of the present disclosure, the XRPD pattern of the above-mentioned crystal form A comprises diffraction peaks as shown in Table 1.

**Table 1. Data for diffraction peaks in XRPD pattern of crystal form A of the compound of formula (I)**

| No. | 2θ [°] | d-Spacing (Å) | Intensity [count] | Relative intensity [%] | No. | 2θ [°] | d-Spacing (Å) | Intensity [count] | Relative intensity [%] |
|---|---|---|---|---|---|---|---|---|---|
| 1 | 7.29 | 12.11821 | 262.024 | 0.30 | 29 | 26.11 | 3.41072 | 933.224 | 3.40 |
| 2 | 8.09 | 10.91653 | 19287 | 100.00 | 30 | 26.50 | 3.36058 | 826.949 | 2.90 |
| 3 | 9.46 | 9.33914 | 3011.45 | 14.60 | 31 | 26.64 | 3.34334 | 969.316 | 3.70 |
| 4 | 9.75 | 9.06181 | 472.462 | 1.40 | 32 | 27.54 | 3.23624 | 380.465 | 0.70 |
| 5 | 11.06 | 7.99650 | 220.323 | 0.30 | 33 | 27.99 | 3.18551 | 1499.56 | 6.60 |
| 6 | 12.10 | 7.31169 | 1016.45 | 4.40 | 34 | 28.32 | 3.14869 | 2189.91 | 10.20 |
| 7 | 12.78 | 6.92315 | 760.608 | 2.90 | 35 | 28.78 | 3.09907 | 358.899 | 0.70 |
| 8 | 13.06 | 6.77489 | 894.541 | 3.60 | 36 | 29.00 | 3.07650 | 593.98 | 2.00 |
| 9 | 13.95 | 6.34547 | 3646.54 | 17.90 | 37 | 30.21 | 2.95575 | 977.517 | 4.10 |
| 10 | 14.87 | 5.95218 | 366.389 | 0.60 | 38 | 30.50 | 2.92882 | 311.454 | 0.70 |
| 11 | 15.28 | 5.79459 | 2923 | 14.10 | 39 | 31.03 | 2.87981 | 210.398 | 0.20 |
| 12 | 16.39 | 5.40461 | 1437.27 | 6.30 | 40 | 31.99 | 2.79562 | 399.362 | 1.30 |
| 13 | 17.12 | 5.17557 | 2892.56 | 13.90 | 41 | 33.33 | 2.68574 | 387.489 | 1.20 |
| 14 | 17.54 | 5.05292 | 497.062 | 1.40 | 42 | 33.69 | 2.65827 | 341.337 | 0.90 |
| 15 | 18.56 | 4.77577 | 607.256 | 1.90 | 43 | 34.01 | 2.63402 | 312.113 | 0.80 |
| 16 | 18.86 | 4.70220 | 997.962 | 3.90 | 44 | 34.39 | 2.60577 | 456.957 | 1.60 |
| 17 | 19.18 | 4.62418 | 4043.84 | 19.80 | 45 | 34.81 | 2.57528 | 314.538 | 0.90 |
| 18 | 19.49 | 4.55020 | 4238.54 | 20.80 | 46 | 35.48 | 2.52837 | 197.476 | 0.30 |
| 19 | 19.84 | 4.47183 | 983.585 | 3.60 | 47 | 35.82 | 2.50489 | 207.026 | 0.30 |
| 20 | 20.82 | 4.26218 | 1561.38 | 6.60 | 48 | 36.37 | 2.46850 | 284.261 | 0.70 |
| 21 | 21.34 | 4.15971 | 4776.22 | 23.50 | 49 | 36.63 | 2.45130 | 222.015 | 0.40 |
| 22 | 22.22 | 3.99818 | 1371.87 | 5.70 | 50 | 37.34 | 2.40628 | 199.047 | 0.40 |
| 23 | 22.96 | 3.87110 | 511.603 | 1.30 | 51 | 37.78 | 2.37912 | 279.458 | 0.80 |
| 24 | 23.58 | 3.76980 | 516.021 | 1.30 | 52 | 38.39 | 2.34280 | 191.966 | 0.30 |
| 25 | 24.11 | 3.68818 | 723.857 | 2.30 | 53 | 38.71 | 2.32405 | 163.344 | 0.20 |
| 26 | 24.74 | 3.59587 | 4338.5 | 21.20 | 54 | 39.14 | 2.29985 | 163.223 | 0.10 |
| 27 | 25.26 | 3.52270 | 4462.29 | 21.80 | 55 | 39.57 | 2.27591 | 174.978 | 0.20 |
| 28 | 25.73 | 3.45952 | 585.892 | 1.50 | | | | | |

In some embodiments of the present disclosure, the above-mentioned crystal form A has a differential scanning calorimetry (DSC) curve comprising an exothermic peak with an onset at 199.55±3.00°C.

In some embodiments of the present disclosure, the above-mentioned crystal form A has a differential scanning calorimetry (DSC) curve comprising an exothermic peak with a peak at 204.65±3.00°C.

In some embodiments of the present disclosure, the above-mentioned crystal form A has a DSC pattern substantially as shown in FIG. 2.

In some embodiments of the present disclosure, the above-mentioned crystal form A has a thermogravimetric analysis (TGA) curve showing a weight loss of 4.502% at 220.00±3.00°C.

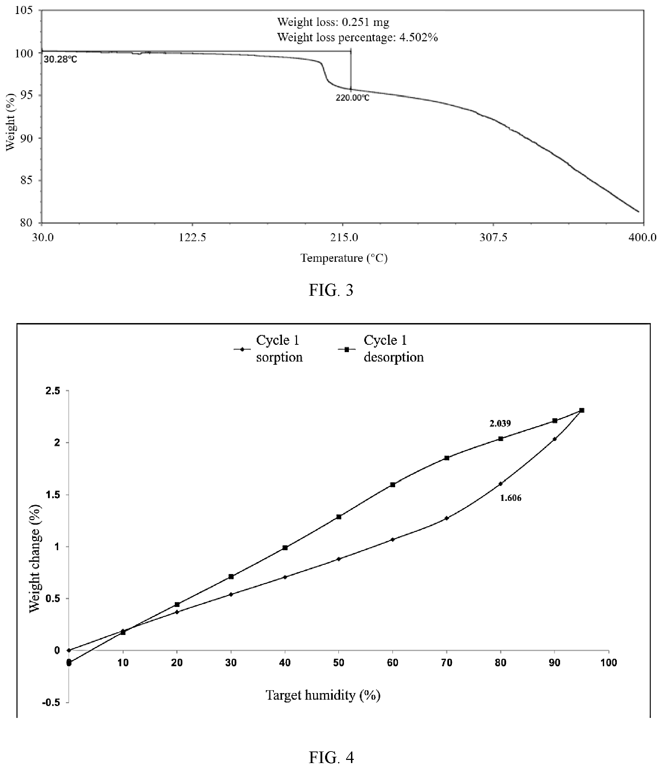

In some embodiments of the present disclosure, the above-mentioned crystal form A has a TGA pattern substantially as shown in FIG. 3.

The present disclosure further provides a pharmaceutical composition comprising the above-mentioned crystal form A of the compound of formula (I).

In some embodiments, the pharmaceutical composition further comprises a pharmaceutically acceptable excipient.

In some embodiments, the pharmaceutical composition comprises a therapeutically effective amount of the above-mentioned crystal form A of the compound of formula (I), and a pharmaceutically acceptable excipient.

The present disclosure further provides a method for treating a KRAS G12D-mutated solid tumor, comprising administering to a subject in need thereof (e.g., a mammal, preferably a human) a therapeutically effective amount of the above-mentioned crystal form A of the compound of formula (I) or the pharmaceutical composition thereof.

The present disclosure further provides the above-mentioned crystal form A of the compound of formula (I) or the pharmaceutical composition thereof for use in treating a KRAS G12D-mutated solid tumor.

The present disclosure further provides a use of the above-mentioned crystal form A of the compound of formula (I) or the pharmaceutical composition thereof in the treatment of a KRAS G12D-mutated solid tumor.

The present disclosure further provides a use of the above-mentioned crystal form A of the compound of formula (I) in the manufacture of a medicament for treating a KRAS G12D-mutated solid tumor.

In some embodiments of the present disclosure, the above-mentioned KRAS G12D-mutated solid tumor is selected from colon cancer and pancreatic cancer.

### Technical effects

The crystal form of the compound of the present disclosure has good stability, favorable hygroscopicity, excellent cell proliferation inhibitory activity against KRAS G12D-mutated tumor cells, effectively inhibits p-ERK, possesses good PK properties, and exhibits significant tumor inhibitory effects.

### Definitions and explanations

Unless otherwise specified, the following terms and phrases used herein are intended to have the following meanings. A specific phrase or term should not be considered indefinite or unclear in the absence of a special definition, but should be understood according to its ordinary meaning. When a trade name appears herein, it is intended to refer to its corresponding commercial product or the active ingredient thereof.

The intermediate compounds of the present disclosure can be prepared by a variety of synthetic methods well known to those skilled in the art, including the specific embodiments enumerated below, embodiments formed by the combination thereof with other chemical synthetic methods, and equivalent replacement methods well known to those skilled in the art. Preferred embodiments include, but are not limited to, the examples of the present disclosure.

The chemical reactions in the specific embodiments of the present disclosure are carried out in a suitable solvent, and the solvent must be suitable for the chemical changes of the present disclosure and the reagents and materials required therefor. In order to obtain the compounds of the present disclosure, it is sometimes necessary for a person skilled in the art to modify or select synthetic steps or reaction processes based on existing embodiments.

Unless otherwise specified, in a powder X-ray diffraction pattern, peak positions or relative intensities of peaks may vary due to factors such as measurement instruments, measurement methods/conditions, and the like. For any specific crystal form, there may be an error in the peak positions, and the measurement error of the 2θ values may be ±0.2°, ±0.15°, and ±0.1°. Therefore, when identifying each crystal form, such error should be taken into account, and those within the error are also encompassed within the scope of the present disclosure.

Unless otherwise specified, a solid wedge bond ( 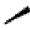 ) and a dashed wedge bond ( 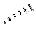 ) are used to indicate the absolute configuration of a stereocenter, and a straight solid bond ( 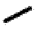 ) and a straight dashed bond ( 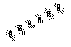 ) are used to indicate the relative configuration of a stereocenter. For example, 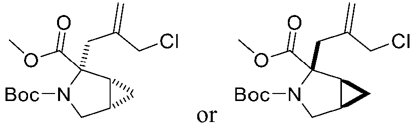 using a straight solid bond ( 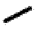 ) and a straight dashed bond ( 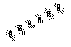 ) to indicate the relative configuration of a stereocenter represents a mixture of 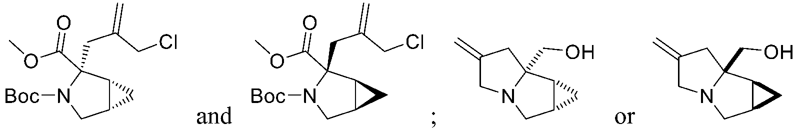 represents a mixture of

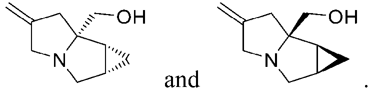

The present disclosure will be specifically described below by way of examples, which are not meant to limit the present disclosure in any way.

The structures of the compounds of the present disclosure can be confirmed by conventional methods well known to those skilled in the art; if the present disclosure involves the absolute configuration of a compound, the absolute configuration can be confirmed by conventional technical means in the art. For example, by single-crystal X-ray diffraction (SXRD), diffraction intensity data of a grown single crystal are collected using a Bruker D8 Venture diffractometer, with a CuKα radiation light source and a (φ/(ω scan mode; after collecting the relevant data, the crystal structure is further resolved by a direct method (Shelxs97) to confirm the absolute configuration.

In the present disclosure, the term "therapeutically effective amount" means an amount of a drug or agent that (i) treats a specific disease, condition, or disorder, or (ii) alleviates, ameliorates, or eliminates one or more symptoms of a specific disease, condition, or disorder. The amount of the crystal form A of the compound of formula (I) of the present disclosure or the pharmaceutical composition thereof that constitutes a "therapeutically effective amount" varies depending on the drug or agent, the disease state and its severity, the mode of administration, and the age of the subject to be treated, but can be routinely determined by a person skilled in the art based on their own knowledge and the contents of the present disclosure.

The term "treating" or "treatment" means administering a drug or agent to ameliorate or eliminate a disease or one or more symptoms associated with said disease, and includes:
(i) inhibiting the disease or disease state, i.e., arresting its development; and
(ii) relieving the disease or disease state, i.e., causing regression of the disease or disease state.

In the present disclosure, the term "subject" includes humans and animals, for example, mammals (such as primates, cattle, horses, pigs, dogs, cats, mice, rats, rabbits, goats, sheep, and avian species, and the like).

The term "pharmaceutically acceptable excipient" refers to those excipients that do not cause significant irritation to an organism and do not impair the biological activity and properties of the active substance. Conventional pharmaceutical excipients include fillers, absorbents, wetting agents, binders, disintegrants, lubricants, carriers, diluents, isotonicity adjusters, pH adjusters, and the like. Suitable excipients are well known to those skilled in the art, such as carbohydrates, waxes, water-soluble and/or water-swellable polymers, hydrophilic or hydrophobic materials, gelatin, oils, solvents, water, and the like.

Typical routes for administering the crystal form A of the compound of formula (I) of the present disclosure or the pharmaceutical composition thereof include, but are not limited to, oral, rectal, topical, inhalation, parenteral, sublingual, intravaginal, intranasal, intraocular, intraperitoneal, intramuscular, subcutaneous, and intravenous administration.

The pharmaceutical composition of the present disclosure can be manufactured by methods well known in the art, such as conventional mixing methods, dissolving methods, granulating methods, emulsifying methods, lyophilizing methods, and the like.

In some embodiments, the pharmaceutical composition may be in an oral form. For oral administration, the pharmaceutical composition can be formulated by mixing the active substance with pharmaceutically acceptable excipients well known in the art. These excipients enable the crystal form A of the compound of formula (I) of the present disclosure or the pharmaceutical composition thereof to be formulated into tablets, pills, lozenges, dragees, capsules, liquids, gels, syrups, slurries, suspensions, and the like, for oral administration to a patient.

Solid oral compositions can be prepared by conventional mixing, filling, or tableting methods. For example, they can be obtained by mixing the active compound with a solid excipient, optionally grinding the resulting mixture, adding other suitable excipients if necessary, and then processing the mixture into granules to obtain cores of tablets or dragees.

The therapeutically effective amount of the crystal form A of the compound of formula (I) contained in the pharmaceutical composition of the present disclosure is selected from 0.001 mg/kg body weight to 1000 mg/kg body weight, for example, 0.01 mg/kg body weight to 500 mg/kg body weight, in the form of a single dose or divided doses.

A person skilled in the art recognizes that the measurement data of XRPD peak positions and/or intensities for a given crystal form of the same compound will vary within an error range. The 2*θ* values in the present disclosure encompass an appropriate error range, which is generally represented by "±". For example, a 2*θ* value represented by a specific angle value ±0.20° in the present disclosure means that the specific angle value therein has an error fluctuation range of ±0.20°; that is, 8.09°±0.2° indicates that 2θ is within the range of 7.89° to 8.29°. Depending on sample preparation techniques, calibration techniques applied to instruments, human operational variations, and the like, a person skilled in the art recognizes that an appropriate error range for XRPD diffraction angles can be ±0.20°, ±0.15°, ±0.10°, ±0.05°, or less, and peak intensities allow for a certain degree of variability. When used to describe an XRPD pattern, the term "substantially the same" or "substantially as shown in..." refers to a pattern including at least 50%, at least 60%, at least 70%, at least 80%, at least 90%, at least 95%, or at least 99% of diffraction peaks whose diffraction angles are within a standard deviation range of ±0.2° 2*θ*.

Since a person skilled in the art recognizes that the measurement data of a DSC pattern for a given crystal form of the same compound will vary within an error tolerance, the peak onset values and single peak maximum values (expressed in degrees Celsius) allow for an appropriate error range. Generally, the error range is represented by "±". For the same crystal form of the same compound, the error in thermal transition temperature and melting point is typically within ±3.00°C in consecutive analyses. For example, a peak value of "204.65±3.00°C" indicates that it is within the range of 201.65°C to 207.65°C. Depending on sample preparation techniques, calibration techniques applied to instruments, human operational variations, and the like, a person skilled in the art recognizes that an appropriate error range for a single peak maximum value can be ±3.0°C, ±2.0°C, or less.

The term "optional" or "optionally" means that the subsequently described event or circumstance may or may not occur, and the description includes instances where the event or circumstance occurs and instances where it does not.

The present disclosure employs the following abbreviations:
ACN represents acetonitrile; DMSO represents dimethyl sulfoxide; MTBE represents methyl tert-butyl ether; N₂ represents nitrogen; RH represents relative humidity; mL represents milliliter; L represents liter; min represents minute; °C represents degrees Celsius; µm represents micrometer; mm represents millimeter; µL represents microliter; mol/L represents moles per liter; mg represents milligram; s represents second; nm represents nanometer; MPa represents megapascal; lux represents lux; µw/cm² represents microwatts per square centimeter; h represents hour; Kg represents kilogram; nM represents nanomolar; 1 M represents 1 mol/L; rpm represents revolutions per minute; XRPD represents X-ray powder diffraction; DSC represents differential scanning calorimetry; TGA represents thermogravimetric analysis; and ¹H NMR represents proton nuclear magnetic resonance.

The compounds of the present disclosure are named according to conventional naming principles in the art or using ChemDraw^{®} software; commercially available compounds employ the supplier catalog names, and all solvents used in the present disclosure are commercially available.

### Instruments and analytical methods

### X-Ray Powder Diffraction (XRPD) method of the present disclosure

Instrument model: Bruker D2 PHASER X-ray powder diffractometer
Testing method: Approximately 10 to 20 mg of sample is used for XRPD detection.

Detailed XRPD parameters are as follows:
Radiation source: Cu, kα, (λ=1.54184 Å).
Tube voltage: 30 kV, tube current: 10 mA
Divergence slit: 1 mm
Detector slit: 0.075 mm
Anti-scatter slit: 3 mm
Scanning range: 3-40 deg
Step size: 0.02 deg
Time per step: 0.3 s

### Single-Crystal X-Ray Diffraction (SXRD) detection and analysis of the present disclosure

Instrument model: Bruker D8 VENTURE
Low-temperature equipment model: Oxford Cryostream 800

### Testing parameters:

Cu rotating anode: λ=1.54184 Å, power: 2.5 kW,
Sample-to-detector distance: d = 45 mm
Voltage: 50 kV
Current: 50 mA
Diffraction experimental temperature: T = 173 K

### Differential Scanning Calorimetry (DSC) method of the present disclosure

Instrument model: Discovery 2500 differential scanning calorimeter
Testing method: A sample (2 to 3 mg) is accurately weighed and placed into a DSC aluminum pan, sealed, and placed on the sample side (S) of the sensor; a blank reference pan is prepared in the same manner and placed on the reference side (R) of the sensor. Under a condition of 50 mL/min N₂, the sample is heated from 30°C (room temperature) to 400°C at a heating rate of 10°C/min.

### Thermogravimetric Analysis (TGA) method of the present disclosure

Instrument model: Discovery 5500 thermogravimetric analyzer
Testing method: An empty pan is placed on the sensor and tared; a sample (5.581 mg) is weighed into the pan using the built-in balance, and tested. Under a condition of 25 mL/min N₂, the sample is heated from 30°C to 400°C at a heating rate of 10°C/min.

### Dynamic Vapor Sorption (DVS) method of the present disclosure

Instrument model: SMS DVS Advantage dynamic vapor sorption analyzer
Testing conditions: A sample (10 to 30 mg) is placed into a DVS sample pan for testing.
Detailed DVS parameters are as follows:
Temperature: 25°C
Equilibrium: dm/dt = 0.005%/min (minimum: 10 min, maximum: 180 min)
Gas: N₂
RH (%) testing steps: 10% (90%-0%-90%); 5% (95%-90%, 90%-95%)
RH (%) testing step range: 0% - 95% - 0%

The classification for evaluating hygroscopicity is as follows:

**Table 2. Classification for evaluating hygroscopicity**

| **Hygroscopicity classification** | ΔW% |
|---|---|
| Deliquescence | Absorbs a sufficient amount of moisture to form a liquid |
| Highly hygroscopic | ΔW% ≥ 15% |
| Hygroscopic | 15% > ΔW% ≥ 2% |
| Slightly hygroscopic | 2% > ΔW% ≥ 0.2% |
| Non-hygroscopic or almost non-hygroscopic | ΔW% < 0.2% |

| | |
|---|---|
| Note: ΔW% represents the hygroscopic weight gain of the test sample under the condition of 25±1°C and 80+/-2% RH. | |

### BRIEF DESCRIPTION OF THE DRAWINGS

FIG. 1 is an XRPD pattern using Cu-Kα radiation of the crystal form A of the compound of formula (I).
FIG. 2 is a DSC pattern of the crystal form A of the compound of formula (I).
FIG. 3 is a TGA pattern of the crystal form A of the compound of formula (I).
FIG. 4 is a DVS pattern of the crystal form A of the compound of formula (I).
FIG. 5 is a stereostructural ellipsoid plot of a single crystal of the compound of formula (I).

### DETAILED DESCRIPTION OF THE PREFERRED EMBODIMENT

T The present disclosure is described in detail below by way of examples, which are not meant to impose any adverse limitation on the present disclosure. The present disclosure has been described in detail herein, including specific embodiments thereof disclosed therein. It will be apparent to a person skilled in the art that various changes and modifications can be made to the specific embodiments of the present disclosure without departing from the spirit and scope of the present disclosure.

### Example 1: Synthesis of the compound of formula (I)

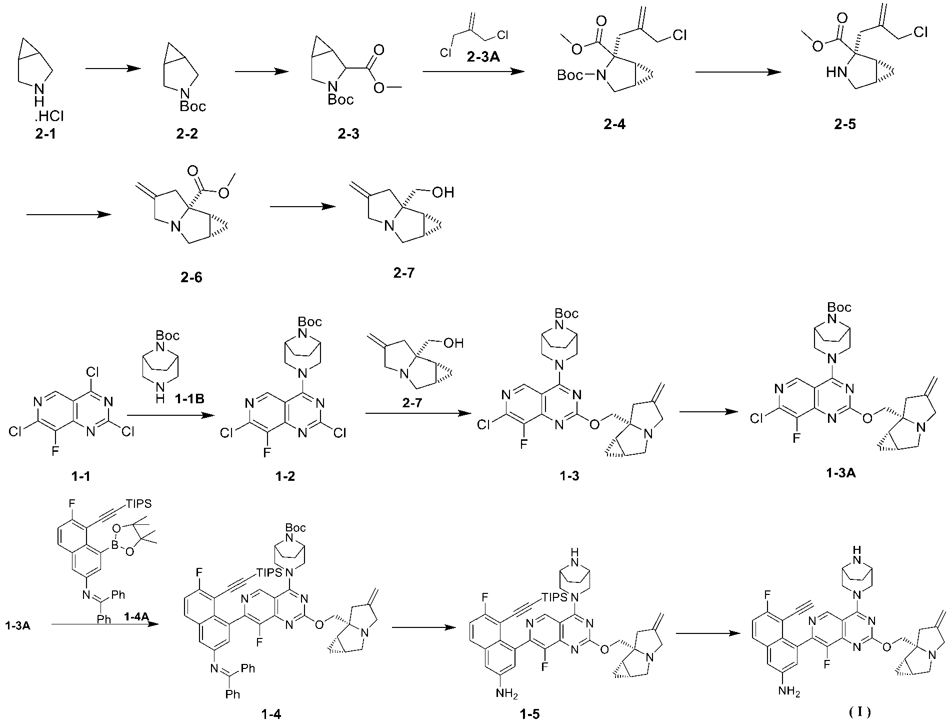

### Step 1: Preparation of intermediate 2-2

Intermediate 2-1 (50 g, 418.09 mmol) was dissolved in dichloromethane (500 mL), and triethylamine (84.61 g, 836.17 mmol) was added thereto, followed by the addition of di-tert-butyl dicarbonate (100.37 g, 459.90 mmol). The reaction was carried out at 25°C for 16 hours. Water (100 mL) was added to the reaction solution, and the phases were separated. The organic phase was dried over anhydrous sodium sulfate, filtered, and concentrated. The crude product was purified by silica gel column chromatography (petroleum ether/ethyl acetate = 100/1 to 50/1) to obtain intermediate **2-2.** MS: m/z = 206.1 [M+Na]⁺.

### Step 2: Preparation of intermediate 2-3

Intermediate **2-2** (25 g, 136.43 mmol) was dissolved in anhydrous tetrahydrofuran (350 mL), and 3,7-dipropyl-3,7-diazabicyclo[3.3.1]nonane (37.31 g, 177.36 mmol) was added thereto. The mixture was cooled to -65°C, and sec-butyllithium (1.3 M, 157.42 mL) was slowly added dropwise. After reacting for 1 hour, methyl chloroformate (15.73 g, 166.44 mmol) was added dropwise, and the reaction was carried out at -65°C for 2 hours. The reaction mixture was quenched by the dropwise addition of saturated ammonium chloride (20 mL), and extracted with ethyl acetate (300 mL * 2). The organic phases were combined, washed with saturated brine (150 mL), dried over anhydrous sodium sulfate, filtered, and concentrated. The crude product was purified by silica gel column chromatography (PE/EA = 50/1 to 25/1) to obtain intermediate **2-3**. MS: m/z = 186.0 [M-tBu+H]⁺.

### Step 3: Preparation of intermediate 2-4

Intermediate **2-3** (12 g, 49.73 mmol) was dissolved in tetrahydrofuran (120 mL), and 3-chloro-2-chloromethylpropene (24.87 g, 198.94 mmol) was added thereto. The mixture was cooled to -40 °C, and lithium bis(trimethylsilyl)amide (1 M, 99.47 mL) was slowly added dropwise. The temperature was gradually raised to 20°C, and the reaction was carried out for 2 hours. The reaction mixture was quenched by the dropwise addition of saturated ammonium chloride (20 mL), and extracted with ethyl acetate (150 mL * 2). The organic phases were combined, washed with saturated brine (80 mL), dried over anhydrous sodium sulfate, filtered, and concentrated. The crude product was purified by silica gel column chromatography (petroleum ether/ethyl acetate = 20/1 to 10/1) to obtain a relative configuration mixture of intermediate **2-4**. TLC thin-layer chromatography (petroleum ether:acetone = 5:1) was developed twice, wherein the Rf value of **2-4** was 0.5, and the Rf value of its isomer was 0.55. The retention time of 2-4 on LCMS (chromatographic column: Agilent Poroshell 120 EC-C18 2.7 micrometers 3.0 * 30 mm; mobile phase: A: water (0.037% formic acid) - B: acetonitrile (0.0187% formic acid); gradient: B: 5%-95%) was 0.776 min, and the retention time of its isomer was 0.801 min. MS: m/z = 274.0 [M-tBu+H]⁺.

### Step 4: Preparation of intermediate 2-5

Intermediate **2-4** (3.6 g, 10.92 mmol) was dissolved in hydrogen chloride/ethyl acetate (4 M, 27.29 mL), and the reaction was carried out at 20°C for 2 hours. The reaction mixture was concentrated under reduced pressure to obtain a crude hydrochloride salt of intermediate **2-5**. MS: m/z = 230.1 [M+H] ⁺.

### Step 5: Preparation of intermediate 2-6

The hydrochloride salt of intermediate **2-5** (2.9 g) was dissolved in methanol (100 mL), and potassium carbonate (4.52 g, 32.69 mmol) was added thereto. The reaction was carried out at 20°C for 2 hours. Dichloromethane (80 mL) was added to the reaction mixture, and the mixture was filtered and concentrated. The crude product was purified by silica gel column chromatography (petroleum ether/ethyl acetate = 10/1 to 5/1) to obtain intermediate **2-6**. MS: m/z = 194.1 [M+H] ⁺.

### Step 6: Preparation of intermediate 2-7

Intermediate **2-6** (1.6 g, 8.28 mmol) was dissolved in anhydrous tetrahydrofuran (20 mL), and lithium aluminum hydride (628.51 mg, 16.56 mmol) was added thereto. The reaction was carried out at 0°C for 2 hours. The reaction mixture was diluted by the dropwise addition of ethyl acetate (10 mL), and water (0.63 mL) was added dropwise. The mixture was dried over anhydrous sodium sulfate, filtered, and concentrated to obtain crude intermediate **2-7**. MS: m/z = 166.1 [M+H] ⁺.

### Step 7: Preparation of intermediate 1-2

Intermediate **1-1** (25 g, 99.03 mmol) was dissolved in dichloromethane (200 mL), and the mixture was cooled to -30°C. Subsequently, N,N-diisopropylethylamine (38.40 g, 297.08 mmol) and **1-1B** (21.02 g, 99.03 mmol) were sequentially added thereto, and the reaction was carried out at -30°C for 2 hours. The reaction mixture was directly concentrated under reduced pressure to obtain crude intermediate **1-2**. MS: m/z = 428.1 [M+1] ⁺.

### Step 8: Preparation of intermediate 1-3A

Intermediate 1-2 (23 g) and intermediate 2-7 (9.76 g) were dissolved in anhydrous toluene (300 mL), and sodium tert-butoxide (13.93 g, 145.00 mmol) was slowly added thereto at 0°C. The reaction was carried out at 0°C for 0.5 hours, and then at 20°C for 0.5 hours. The reaction mixture was diluted by adding ethyl acetate (200 mL), washed with saturated brine (100 mL), dried over anhydrous sodium sulfate, and filtered. The filtrate was concentrated under reduced pressure to obtain a crude product **1-3**. The crude product **1-3** was purified by preparative SFC (chromatographic column: DAICEL CHIRALCEL OD (250 mm * 50 mm, 10 micrometers); mobile phase: [A (supercritical carbon dioxide), B (ethanol containing 0.1% aqueous ammonia)]; B: 40%) to obtain a single isomer **1-3A** and its isomer. The retention time of **1-3A** under analytical SFC conditions (chromatographic column: Cellulose 2 100 mm * 4.6 mm, 3 micrometers; mobile phase: [A (supercritical carbon dioxide), B (methanol (0.05% diethylamine))]; B: 40%) was 4.964 min, with an ee value of 95.7%. ¹H NMR (400MHz, CD₃OD) δ 8.76 (s, 1H), 4.98 - 4.87 (m, 2H), 4.51 - 4.48 (m, 3H), 4.34 - 4.29 (m, 1H), 4.27 (br s, 2H), 4.23 - 4.18 (m, 1H), 3.61 - 3.58 (m, 3H), 3.12 (d, J=9.5 Hz, 1H), 2.78 (br d, J=16.8 Hz, 1H), 2.71 (dd, J=4.0, 9.6 Hz, 1H), 2.45 (br d, J=16.8 Hz, 1H), 1.83 - 1.72 (m, 2H), 1.75 - 1.62 (m, 3H), 1.55 - 1.51 (m, 1H), 1.42 (s, 9H), 0.60 (q, J=4.2 Hz, 1H), 0.47 -0.42 (m, 1H). MS: m/z = 557.2 [M+1]⁺. Its isomer had a retention time of 8.382 min under the same conditions, with an ee value of 96.5%.

### Step 9: Synthesis of intermediate 1-4

Intermediate **1-3A** (11.3 g, 20.29 mmol) was added into water (60 mL) and anhydrous dioxan (240 mL), and then methanesulfonato(diadamantyl-n-butylphosphino) (2'-amino-1,1'-biphenyl-2-yl)palladium(II) (1.48 g, 2.03 mmol), intermediate **1-4A** (15.38 g, 24.34 mmol), and cesium carbonate (13.22 g, 40.57 mmol) were added thereto. The reaction was carried out at 87°C for 2 hours. The reaction mixture was diluted by adding ethyl acetate (200 mL), washed with saturated brine (40 mL * 2), dried over anhydrous sodium sulfate, and filtered. The filtrate was concentrated under reduced pressure to obtain a crude product. The crude product was purified by column chromatography (mobile phase: dichloromethane:methanol = 50:1 to 20:1) to obtain intermediate 1-4. MS (ESI) m/z: 862.4 [M+H₂O-Ph₂CO+1]⁺.

### Step 10: Synthesis of intermediate 1-5

Intermediate **1-4** (8.4 g, 8.18 mmol) was added into ethyl acetate (30 mL) and water (10 mL), and then hydrogen chloride/ethyl acetate (4 M, 62.37 mL) was added thereto. The reaction was carried out at 20°C for 2 hours. Water (30 mL) was added to the reaction mixture, and the organic phase was washed with water (30 mL * 2). The aqueous phases were combined, and the pH thereof was adjusted to 7-8 with saturated sodium bicarbonate, followed by extraction with ethyl acetate (80 mL * 3). The organic phases were combined, dried over anhydrous sodium sulfate, and filtered. The filtrate was concentrated under reduced pressure to obtain a crude product **1-5**. MS (ESI) m/z: 762.3 [M+1]⁺.

### Step 11: Synthesis of the compound of formula (I)

Intermediate **1-5** (6.2 g) was dissolved in acetonitrile (70 mL), and then tetramethylammonium fluoride (2.29 g, 13.83 mmol) was added thereto. The reaction was carried out at 60 °C for 1 hour. The reaction mixture was diluted by adding ethyl acetate (100 mL), washed with saturated sodium bicarbonate (30 mL), dried over anhydrous sodium sulfate, and filtered. The filtrate was concentrated under reduced pressure to obtain a crude product. The crude product was purified by column chromatography (mobile phase: dichloromethane (10% solution of ammonia): methanol = 20:1 to 10:1) to obtain the **compound of formula (I)**. MS (ESI) m/z: 606.3 [M+1]⁺, ¹H NMR (400 MHz, CD₃OD) δ = 9.01 (s, 1H), 7.76 (dd, J = 5.6, 9.2 Hz, 1H), 7.28 - 7.19 (m, 2H), 7.15 (d, J= 2.3 Hz, 1H), 5.08 (br s, 1H), 5.01 (br s, 1H), 4.70 - 4.54 (m, 3H), 4.43 (dd, J = 7.3, 10.0 Hz, 1H), 4.32 (dd, J = 6.3,10.3 Hz, 1H), 3.78 - 3.65 (m, 5H), 3.39 - 3.34 (m, 1H), 3.23 (d, J = 9.5 Hz, 1H), 2.93 (br d, J = 17.3 Hz, 1H), 2.82(dd, J = 4.0, 9.3 Hz, 1H), 2.56 (br d, J = 17.1 Hz, 1H), 1.91 - 1.76 (m, 5H), 1.64 (td, J = 3.6, 6.9 Hz, 1H), 0.72 (q, J =4.0 Hz, 1H), 0.61 - 0.53 (m, 1H).

### Example 2-1: Preparation of crystal form A of the compound of formula (I)

The compound of formula (I) (37 g) was added into 250 mL of methyl tert-butyl ether, and the mixture was suspended and stirred at 45°C for 4 hours, and then stirred at 20°C for 16 hours. The mixture was filtered, and the filter cake was rinsed with methyl tert-butyl ether (30 mL). The filter cake was dried under reduced pressure to obtain the crystal form A of the compound of formula (I) as a solid. Its XRPD pattern is shown in FIG. 1, its DSC pattern is shown in FIG. 2, and its TGA pattern is shown in FIG. 3.

### Example 2-2: Preparation of crystal form A of the compound of formula (I)

The compound of formula (I) (34 g) was added into 60 mL of ethanol, and heated to dissolve at 45°C. 40 ML of methyl tert-butyl ether was slowly added thereto, followed by the addition of seeds of crystal form A for induction. The mixture was stirred at 45°C for 2 hours, and then 200 mL of methyl tert-butyl ether was slowly added thereto. The mixture was first stirred at 45°C for 1 hour, and then cooled to 20°C and stirred for 16 hours. The mixture was filtered, and the filter cake was rinsed with 20 mL of n-heptane, and dried to obtain the crystal form A of the compound of formula (I). Its XRPD pattern is substantially identical to FIG. 1, its DSC pattern is substantially identical to FIG. 2, and its TGA pattern is substantially identical to FIG. 3.

### Example 2-3: Preparation of crystal form of the compound of formula (I)

10 Mg of the compound of formula (I) was added into 2 mL of ethanol, and the mixture was stirred. The resulting sample appeared insoluble. 0.5 ML of methanol was then added dropwise thereto, and the mixture was stirred until the sample was completely dissolved to obtain a clear yellow solution. The sample solution was placed into a 4 mL semi-sealed vial, and allowed to slowly evaporate at room temperature. Colorless block crystals were obtained after one week.

### Example 3: Hygroscopicity study of crystal form A of the compound of formula (I)

### Experimental materials:

SMS DVS Intrinsic dynamic vapor sorption analyzer

### Experimental method:

10 to 30 mg of the crystal form A of the compound of formula (I) was placed into a DVS sample pan for testing.

### Experimental results:

The DVS pattern of the crystal form A of the compound of formula (I) is shown in FIG. 4. When the humidity was increased to 80%, ΔW = 1.606%.

### Experimental conclusion:

The hygroscopic weight gain of the crystal form A of the compound of formula (I) at 25°C and 80% RH was 1.606%, indicating that the sample is slightly hygroscopic.

### Example 4: Single-crystal X-ray diffraction detection and analysis of the compound of formula (I)

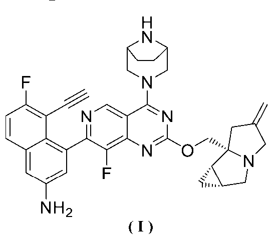

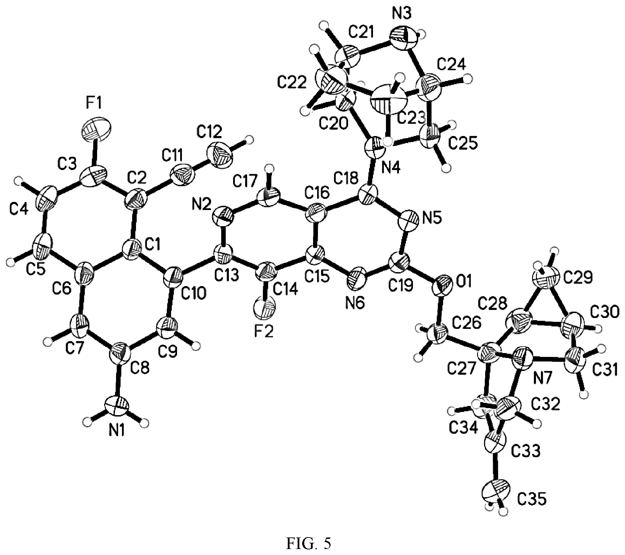

The colorless block crystals obtained in Example 2-3 were taken, and the diffraction intensity data were collected using a single-crystal X-ray diffractometer. The basic structural information of the compound is as follows: molecular formula: C₃₅H₃₃F₂N₇O, monoclinic system, space group P2₁. The unit cell parameters are: a = 7.9101(4) Å, b = 17.6683(9) Å, c = 10.7175(6) Å, *α* =γ *=* 90°, *β* = 95.763(3)°, and the volume V = 1490.29(14) Å³. The absolute configuration parameter Flack parameter is 0.16(17). The single-crystal data can determine the absolute configuration of the compound of formula (I). The stereostructural ellipsoid plot of the single crystal of the compound of formula (I) is shown in FIG. 5. The crystal structure data and parameters of the compound of formula (I) are shown in Tables 3, 4, 5, 6, 7, and 8.

**Table 3. Crystal data of the single crystal of the compound of formula (I)**

| | | |
|---|---|---|
| Formula | | C₃₅H₃₃F₂N₇O |
| Crystal size | | 0.140 x 0.120 x 0.100 mm³ |
| Crystal system | | Monoclinic |
| Space group | | P2(1) |
| Unit cell dimensions | a/Å | 7.9101(4) |
| | b/Å | 17.6683(9) |
| | c/Å | 10.7175(6) |
| Volume(Å³) | | 1490.29(14) |
| Temperature | | 173(2) K |
| Wavelength | | 1.54178 A |
| Z, Calculated density | | 2, 1.350 mg/m³ |
| Absorption coefficient | | 0.762 mm⁻¹ |
| F(000) | | 636 |
| Theta range for data collection | | 4.146 to 66.582 deg. |
| Limiting indices | | -9<=h<=9, -21<=k<=20, - 12<=l<=12 |
| Completeness to theta = 66.582 | | 99.9 % |
| Absorption correction | | Semi-empirical from equivalents |
| Max. and min. transmission | | 0.7531 and 0.6467 |
| Goodness-of-fit on F^2 | | 1.015 |
| Final R indices [I>2sigma(I)] | | R1 = 0.0519, wR2 = 0.1187 |
| R indices (all data) | | R1 = 0.0810, wR2 = 0.1363 |
| Absolute structure parameter | | 0.16(17) |
| Largest diff. peak and hole | | 0.201 and -0.196 e.A⁻³ |

**Table 4. Atomic coordinates (x 10⁴) and equivalent isotropic displacement parameters (Å² × 10³) of the crystal of the compound of formula (I)**

| Atom | x | y | z | U(eq) |
|---|---|---|---|---|
| N(1) | 3414(6) | 8409(3) | 6883(5) | 38(1) |
| C(1) | 6789(6) | 6545(3) | 6907(5) | 28(1) |
| F(2) | 3437(4) | 6250(2) | 3953(3) | 37(1) |
| N(2) | 4223(5) | 5200(2) | 6848(4) | 30(1) |
| C(2) | 8028(7) | 5959(3) | 6834(5) | 32(1) |
| N(3) | 2286(6) | 1587(3) | 6674(5) | 40(1) |
| C(3) | 9615(7) | 6064(3) | 7454(6) | 38(1) |
| N(4) | 1769(6) | 3110(2) | 5623(4) | 31(1) |
| C(4) | 10091(7) | 6729(3) | 8111(6) | 38(1) |
| N(5) | 498(5) | 3771(2) | 3919(4) | 29(1) |
| C(5) | 8991(7) | 7307(3) | 8105(5) | 37(1) |
| N(6) | 1415(5) | 5014(2) | 3330(4) | 30(1) |
| C(6) | 7311(6) | 7244(3) | 7505(5) | 30(1) |
| N(7) | -4067(6) | 4526(2) | 980(4) | 33(1) |
| C(7) | 6172(7) | 7860(3) | 7489(5) | 34(1) |
| C(8) | 4526(7) | 7806(3) | 6943(5) | 30(1) |
| C(9) | 3999(7) | 7094(3) | 6430(5) | 30(1) |
| C(10) | 5048(6) | 6486(3) | 6406(5) | 27(1) |
| C(11) | 7749(7) | 5291(3) | 6062(6) | 35(1) |
| C(12) | 7553(8) | 4757(4) | 5396(7) | 50(2) |
| F(1) | 10777(4) | 5508(2) | 7436(4) | 54(1) |
| O(1) | -742(5) | 4356(2) | 2228(3) | 33(1) |
| C(13) | 4256(6) | 5754(3) | 5961(5) | 27(1) |
| C(14) | 3417(6) | 5679(3) | 4804(5) | 28(1) |
| C(15) | 2447(6) | 5025(3) | 4440(5) | 26(1) |
| C(16) | 2568(6) | 4429(3) | 5312(5) | 27(1) |
| C(17) | 3416(7) | 4566(3) | 6516(5) | 31(1) |
| C(18) | 1603(6) | 3755(3) | 4927(5) | 27(1) |
| C(19) | 460(7) | 4407(3) | 3217(5) | 29(1) |
| C(20) | 3463(6) | 2809(3) | 6088(5) | 32(1) |
| C(21) | 3295(8) | 2242(3) | 7136(6) | 38(1) |
| C(22) | 2231(8) | 2557(4) | 8138(6) | 47(2) |
| C(23) | 390(8) | 2325(4) | 7658(6) | 48(2) |
| C(24) | 584(8) | 1934(3) | 6413(6) | 41(1) |
| C(25) | 499(7) | 2505(3) | 5331(5) | 34(1) |
| C(26) | -1121(7) | 5042(3) | 1503(5) | 32(1) |
| C(27) | -2538(7) | 4855(3) | 476(5) | 33(1) |
| C(28) | -2074(8) | 4279(4) | -475(6) | 43(2) |
| C(29) | -2175(9) | 3453(4) | -130(6) | 49(2) |
| C(30) | -3668(8) | 3827(4) | -845(6) | 47(2) |
| C(31) | -5018(8) | 4140(3) | -105(6) | 42(2) |
| C(32) | -5003(8) | 5184(3) | 1409(6) | 40(1) |
| C(33) | -4761(7) | 5812(3) | 479(6) | 40(1) |
| C(34) | -3201(8) | 5607(3) | -152(6) | 42(2) |
| C(35) | -5758(9) | 6401(4) | 238(7) | 56(2) |

**Table 5. Bond lengths (Length/Å) of the crystal of the compound of formula (I)**

| Bond | Bond length [Å] | Bond | Bond Length [Å] |
|---|---|---|---|
| N(1)-C(8) | 1.380(7) | C(8)-C(9) | 1.419(7) |
| C(1)-C(10) | 1.431(7) | C(9)-C(10) | 1.359(7) |
| C(1)-C(6) | 1.433(7) | C(10)-C(13) | 1.495(7) |
| C(1)-C(2) | 1.434(7) | C(11)-C(12) | 1.184(9) |
| F(2)-C(14) | 1.361(6) | O(1)-C(19) | 1.354(6) |
| N(2)-C(17) | 1.320(7) | O(1)-C(26) | 1.455(6) |
| N(2)-C(13) | 1.366(7) | C(13)-C(14) | 1.353(7) |
| C(2)-C(3) | 1.373(8) | C(14)-C(15) | 1.419(7) |
| C(2)-C(11) | 1.445(8) | C(15)-C(16) | 1.404(7) |
| N(3)-C(21) | 1.463(8) | C(16)-C(17) | 1.414(7) |
| N(3)-C(24) | 1.480(8) | C(16)-C(18) | 1.453(7) |
| C(3)-F(1) | 1.346(7) | C(20)-C(21) | 1.520(8) |
| C(3)-C(4) | 1.402(9) | C(21)-C(22) | 1.534(9) |
| N(4)-C(18) | 1.361(7) | C(22)-C(23) | 1.550(9) |
| N(4)-C(25) | 1.478(7) | C(23)-C(24) | 1.524(9) |
| N(4)-C(20) | 1.481(7) | C(24)-C(25) | 1.533(8) |
| C(4)-C(5) | 1.341(8) | C(26)-C(27) | 1.526(7) |
| N(5)-C(18) | 1.320(6) | C(27)-C(28) | 1.511(8) |
| N(5)-C(19) | 1.351(7) | C(27)-C(34) | 1.556(8) |
| C(5)-C(6) | 1.421(7) | C(28)-C(29) | 1.510(9) |
| N(6)-C(19) | 1.311(7) | C(28)-C(30) | 1.511(9) |
| N(6)-C(15) | 1.373(6) | C(29)-C(30) | 1.496(8) |
| C(6)-C(7) | 1.411(8) | C(30)-C(31) | 1.498(10) |
| N(7)-C(32) | 1.476(7) | C(32)-C(33) | 1.516(9) |
| N(7)-C(31) | 1.486(7) | C(33)-C(35) | 1.316(8) |
| N(7)-C(27) | 1.491(7) | C(33)-C(34) | 1.509(9) |
| C(7)-C(8) | 1.376(8) | | |

**Table 6. Bond angles (Angle/°) of the single crystal of the compound of formula (I)**

| Bond | Bond angle [deg] | Bond | Bond angle [deg] |
|---|---|---|---|
| C(10)-C(1)-C(6) | 117.0(5) | C(16)-C(15)-C(14) | 115.3(4) |
| C(10)-C(1)-C(2) | 124.4(5) | C(15)-C(16)-C(17) | 118.3(5) |
| C(6)-C(1)-C(2) | 118.6(4) | C(15)-C(16)-C(18) | 115.3(4) |
| C(17)-N(2)-C(13) | 117.6(5) | C(17)-C(16)-C(18) | 125.9(5) |
| C(3)-C(2)-C(1) | 118.2(5) | N(2)-C(17)-C(16) | 124.2(5) |
| C(3)-C(2)-C(11) | 117.8(5) | N(5)-C(18)-N(4) | 119.1(5) |
| C(1)-C(2)-C(11) | 123.7(5) | N(5)-C(18)-C(16) | 120.0(5) |
| C(21)-N(3)-C(24) | 101.1(4) | N(4)-C(18)-C(16) | 120.8(4) |
| F(1)-C(3)-C(2) | 119.2(5) | N(6)-C(19)-N(5) | 130.4(5) |
| F(1)-C(3)-C(4) | 118.0(5) | N(6)-C(19)-O(1) | 118.7(5) |
| C(2)-C(3)-C(4) | 122.9(5) | N(5)-C(19)-O(1) | 110.8(4) |
| C(18)-N(4)-C(25) | 117.6(4) | N(4)-C(20)-C(21) | 109.9(4) |
| C(18)-N(4)-C(20) | 121.2(4) | N(3)-C(21)-C(20) | 110.9(5) |
| C(25)-N(4)-C(20) | 112.6(4) | N(3)-C(21)-C(22) | 101.8(5) |
| C(5)-C(4)-C(3) | 119.7(5) | C(20)-C(21)-C(22) | 111.9(5) |
| C(18)-N(5)-C(19) | 116.8(4) | C(21)-C(22)-C(23) | 103.6(5) |
| C(4)-C(5)-C(6) | 121.3(5) | C(24)-C(23)-C(22) | 103.7(5) |
| C(19)-N(6)-C(15) | 112.3(4) | N(3)-C(24)-C(23) | 101.1(5) |
| C(7)-C(6)-C(5) | 120.8(5) | N(3)-C(24)-C(25) | 112.6(5) |
| C(7)-C(6)-C(1) | 120.2(4) | C(23)-C(24)-C(25) | 111.3(5) |
| C(5)-C(6)-C(1) | 118.9(5) | N(4)-C(25)-C(24) | 109.8(4) |
| C(32)-N(7)-C(31) | 112.0(4) | O(1)-C(26)-C(27) | 107.6(4) |
| C(32)-N(7)-C(27) | 104.9(4) | N(7)-C(27)-C(28) | 104.0(5) |
| C(31)-N(7)-C(27) | 105.1(5) | N(7)-C(27)-C(26) | 112.7(4) |
| C(8)-C(7)-C(6) | 121.8(5) | C(28)-C(27)-C(26) | 114.9(5) |
| C(7)-C(8)-N(1) | 122.5(5) | N(7)-C(27)-C(34) | 103.8(5) |
| C(7)-C(8)-C(9) | 117.1(5) | C(28)-C(27)-C(34) | 112.2(5) |
| N(1)-C(8)-C(9) | 120.4(5) | C(26)-C(27)-C(34) | 108.6(5) |
| C(10)-C(9)-C(8) | 123.5(5) | C(29)-C(28)-C(27) | 117.7(5) |
| C(9)-C(10)-C(1) | 120.1(5) | C(29)-C(28)-C(30) | 59.4(4) |
| C(9)-C(10)-C(13) | 116.9(4) | C(27)-C(28)-C(30) | 106.3(5) |
| C(1)-C(10)-C(13) | 122.7(4) | C(30)-C(29)-C(28) | 60.4(4) |
| C(12)-C(11)-C(2) | 177.5(7) | C(29)-C(30)-C(31) | 117.4(6) |
| C(19)-O(1)-C(26) | 117.0(4) | C(29)-C(30)-C(28) | 60.3(4) |
| C(14)-C(13)-N(2) | 121.7(5) | C(31)-C(30)-C(28) | 106.7(5) |
| C(14)-C(13)-C(10) | 121.9(5) | N(7)-C(31)-C(30) | 104.6(5) |
| N(2)-C(13)-C(10) | 115.8(4) | N(7)-C(32)-C(33) | 105.7(5) |
| C(13)-C(14)-F(2) | 120.2(4) | C(35)-C(33)-C(34) | 127.1(7) |
| C(13)-C(14)-C(15) | 122.2(5) | C(35)-C(33)-C(32) | 126.5(7) |
| F(2)-C(14)-C(15) | 117.5(4) | C(34)-C(33)-C(32) | 106.3(5) |
| N(6)-C(15)-C(16) | 124.1(4) | C(33)-C(34)-C(27) | 105.5(5) |
| N(6)-C(15)-C(14) | 120.5(4) | | |

**Table 7. Torsion angles (Angle/°) of the single crystal of the compound of formula (I)**

| A-B-C-D | Angle/° | A-B-C-D | Angle/° |
|---|---|---|---|
| C(10)-C(1)-C(2)-C(3) | -174.1(5) | C(15)-C(16)-C(18)-N(4) | 172.4(5) |
| C(6)-C(1)-C(2)-C(3) | 6.7(8) | C(17)-C(16)-C(18)-N(4) | -16.0(8) |
| C(10)-C(1)-C(2)-C(11) | 11.1(9) | C(15)-N(6)-C(19)-N(5) | -9.7(8) |
| C(6)-C(1)-C(2)-C(11) | -168.1(5) | C(15)-N(6)-C(19)-O(1) | 173.5(5) |
| C(1)-C(2)-C(3)-F(1) | 177.5(5) | C(18)-N(5)-C(19)-N(6) | 4.0(9) |
| C(11)-C(2)-C(3)-F(1) | -7.4(8) | C(18)-N(5)-C(19)-O(1) | -178.9(4) |
| C(1)-C(2)-C(3)-C(4) | -2.6(9) | C(26)-O(1)-C(19)-N(6) | -12.2(7) |
| C(11)-C(2)-C(3)-C(4) | 172.5(5) | C(26)-O(1)-C(19)-N(5) | 170.4(4) |
| F(1)-C(3)-C(4)-C(5) | 177.2(5) | C(18)-N(4)-C(20)-C(21) | 163.0(5) |
| C(2)-C(3)-C(4)-C(5) | -2.7(9) | C(25)-N(4)-C(20)-C(21) | -49.9(6) |
| C(3)-C(4)-C(5)-C(6) | 3.6(9) | C(24)-N(3)-C(21)-C(20) | -68.6(6) |
| C(4)-C(5)-C(6)-C(7) | -179.0(6) | C(24)-N(3)-C(21)-C(22) | 50.6(5) |
| C(4)-C(5)-C(6)-C(1) | 0.6(8) | N(4)-C(20)-C(21)-N(3) | 63.2(6) |
| C(10)-C(1)-C(6)-C(7) | -5.4(8) | N(4)-C(20)-C(21)-C(22) | -49.7(6) |
| C(2)-C(1)-C(6)-C(7) | 173.8(5) | N(3)-C(21)-C(22)-C(23) | -29.1(6) |
| C(10)-C(1)-C(6)-C(5) | 175.0(5) | C(20)-C(21)-C(22)-C(23) | 89.4(6) |
| C(2)-C(1)-C(6)-C(5) | -5.8(8) | C(21)-C(22)-C(23)-C(24) | -2.3(6) |
| C(5)-C(6)-C(7)-C(8) | -178.2(5) | C(21)-N(3)-C(24)-C(23) | -52.0(5) |
| C(1)-C(6)-C(7)-C(8) | 2.1(8) | C(21)-N(3)-C(24)-C(25) | 66.7(6) |
| C(6)-C(7)-C(8)-N(1) | -177.9(5) | C(22)-C(23)-C(24)-N(3) | 32.4(6) |
| C(6)-C(7)-C(8)-C(9) | 2.0(8) | C(22)-C(23)-C(24)-C(25) | -87.4(6) |
| C(7)-C(8)-C(9)-C(10) | -3.0(9) | C(18)-N(4)-C(25)-C(24) | -164.3(5) |
| N(1)-C(8)-C(9)-C(10) | 176.9(5) | C(20)-N(4)-C(25)-C(24) | 47.3(6) |
| C(8)-C(9)-C(10)-C(1) | -0.4(8) | N(3)-C(24)-C(25)-N(4) | -58.2(7) |
| C(8)-C(9)-C(10)-C(13) | 173.5(5) | C(23)-C(24)-C(25)-N(4) | 54.4(6) |
| C(6)-C(1)-C(10)-C(9) | 4.5(8) | C(19)-O(1)-C(26)-C(27) | -178.2(4) |
| C(2)-C(1)-C(10)-C(9) | -174.6(5) | C(32)-N(7)-C(27)-C(28) | -153.8(4) |
| C(6)-C(1)-C(10)-C(13) | -169.1(5) | C(31)-N(7)-C(27)-C(28) | -35.6(5) |
| C(2)-C(1)-C(10)-C(13) | 11.8(8) | C(32)-N(7)-C(27)-C(26) | 81.0(5) |
| C(17)-N(2)-C(13)-C(14) | 3.6(8) | C(31)-N(7)-C(27)-C(26) | - 160.7(4) |
| C(17)-N(2)-C(13)-C(10) | 174.8(5) | C(32)-N(7)-C(27)-C(34) | -36.2(5) |
| C(9)-C(10)-C(13)-C(14) | 59.1(7) | C(31)-N(7)-C(27)-C(34) | 82.0(5) |
| C(1)-C(10)-C(13)-C(14) | -127.1(6) | O(1)-C(26)-C(27)-N(7) | 55.0(6) |
| C(9)-C(10)-C(13)-N(2) | -112.1(5) | O(1)-C(26)-C(27)-C(28) | -63.9(6) |
| C(1)-C(10)-C(13)-N(2) | 61.7(7) | O(1)-C(26)-C(27)-C(34) | 169.4(5) |
| N(2)-C(13)-C(14)-F(2) | 178.2(5) | N(7)-C(27)-C(28)-C(29) | -41.6(7) |
| C(10)-C(13)-C(14)-F(2) | 7.5(8) | C(26)-C(27)-C(28)-C(29) | 82.1(7) |
| N(2)-C(13)-C(14)-C(15) | 0.3(8) | C(34)-C(27)-C(28)-C(29) | -153.1(5) |
| C(10)-C(13)-C(14)-C(15) | -170.3(5) | N(7)-C(27)-C(28)-C(30) | 21.9(6) |
| C(19)-N(6)-C(15)-C(16) | 4.7(7) | C(26)-C(27)-C(28)-C(30) | 145.6(5) |
| C(19)-N(6)-C(15)-C(14) | -172.1(5) | C(34)-C(27)-C(28)-C(30) | -89.7(6) |
| C(13)-C(14)-C(15)-N(6) | 170.4(5) | C(27)-C(28)-C(29)-C(30) | 93.3(6) |
| F(2)-C(14)-C(15)-N(6) | -7.6(7) | C(28)-C(29)-C(30)-C(31) | -94.3(6) |
| C(13)-C(14)-C(15)-C(16) | -6.6(7) | C(27)-C(28)-C(30)-C(29) | -112.9(6) |
| F(2)-C(14)-C(15)-C(16) | 175.4(4) | C(29)-C(28)-C(30)-C(31) | 112.5(6) |
| N(6)-C(15)-C(16)-C(17) | -168.0(5) | C(27)-C(28)-C(30)-C(31) | -0.5(7) |
| C(14)-C(15)-C(16)-C(17) | 8.9(7) | C(32)-N(7)-C(31)-C(30) | 148.8(5) |
| N(6)-C(15)-C(16)-C(18) | 4.3(7) | C(27)-N(7)-C(31)-C(30) | 35.5(6) |
| C(14)-C(15)-C(16)-C(18) | -178.8(5) | C(29)-C(30)-C(31)-N(7) | 43.3(7) |
| C(13)-N(2)-C(17)-C(16) | -0.8(8) | C(28)-C(30)-C(31)-N(7) | -21.3(6) |
| C(15)-C(16)-C(17)-N(2) | -5.7(8) | C(31)-N(7)-C(32)-C(33) | -77.5(6) |
| C(18)-C(16)-C(17)-N(2) | -177.1(5) | C(27)-N(7)-C(32)-C(33) | 35.9(5) |
| C(19)-N(5)-C(18)-N(4) | -175.9(5) | N(7)-C(32)-C(33)-C(35) | 156.6(6) |
| C(19)-N(5)-C(18)-C(16) | 6.8(7) | N(7)-C(32)-C(33)-C(34) | -20.9(6) |
| C(25)-N(4)-C(18)-N(5) | -10.0(7) | C(35)-C(33)-C(34)-C(27) | -179.0(6) |
| C(20)-N(4)-C(18)-N(5) | 135.5(5) | C(32)-C(33)-C(34)-C(27) | -1.5(6) |
| C(25)-N(4)-C(18)-C(16) | 167.3(5) | N(7)-C(27)-C(34)-C(33) | 23.0(6) |
| C(20)-N(4)-C(18)-C(16) | -47.1(7) | C(28)-C(27)-C(34)-C(33) | 134.7(5) |
| C(15)-C(16)-C(18)-N(5) | -10.3(7) | C(26)-C(27)-C(34)-C(33) | -97.2(5) |
| C(17)-C(16)-C(18)-N(5) | 161.3(5) | | |

**Table 8. Hydrogen atom coordinates (x 10⁴) and isotropic displacement parameters (Å² ×10³) of the single crystal of the compound of formula (I)**

| | x | y | z | U (equivalent) |
|---|---|---|---|---|
| H(1A) | 3749 | 8850 | 7201 | 45 |
| H(1B) | 2370 | 8354 | 6526 | 45 |
| H(1) | 2530(100) | 1420(40) | 5950(70) | 60 |
| H(4) | 11187 | 6770 | 8557 | 45 |
| H(5) | 9342 | 7767 | 8510 | 45 |
| H(7) | 6553 | 8325 | 7865 | 41 |
| H(9) | 2852 | 7039 | 6085 | 36 |
| H(12) | 7397 | 4328 | 4862 | 60 |
| H(17) | 3403 | 4177 | 7126 | 37 |
| H(20A) | 4213 | 3230 | 6404 | 38 |
| H(20B) | 3983 | 2560 | 5393 | 38 |
| H(21) | 4440 | 2079 | 7522 | 46 |
| H(22A) | 2341 | 3115 | 8202 | 56 |
| H(22B) | 2580 | 2330 | 8968 | 56 |
| H(23A) | -89 | 1975 | 8253 | 57 |
| H(23B) | -355 | 2775 | 7535 | 57 |
| H(24) | -303 | 1533 | 6242 | 49 |
| H(25A) | 729 | 2244 | 4548 | 41 |
| H(25B) | -653 | 2728 | 5203 | 41 |
| H(26A) | -97 | 5217 | 1127 | 38 |
| H(26B) | -1491 | 5448 | 2050 | 38 |
| H(28) | -1338 | 4435 | -1136 | 51 |
| H(29A) | -1513 | 3088 | -586 | 59 |
| H(29B) | -2264 | 3326 | 761 | 59 |
| H(30) | -3966 | 3694 | -1747 | 56 |
| H(31A) | -5750 | 4503 | -614 | 51 |
| H(31B) | -5737 | 3729 | 181 | 51 |
| H(32A) | -4541 | 5336 | 2265 | 49 |
| H(32B) | -6224 | 5063 | 1416 | 49 |
| H(34A) | -2331 | 6009 | -21 | 50 |
| H(34B) | -3494 | 5535 | -1064 | 50 |
| H(35A) | -5523 | 6752 | -393 | 68 |
| H(35B) | -6713 | 6476 | 696 | 68 |

### Example 5: Solid stability test of crystal form A of the compound of formula (I)

According to the "Guidelines for the Stability Testing of Drug Substances and Pharmaceutical Preparations" (General Chapter 9001, Part IV, Chinese Pharmacopoeia 2020 Edition), the solid stability of crystal form A of the compound of formula (I) was evaluated. The crystal form A was respectively placed under conditions of high temperature (60°C, open), high humidity (25°C / 92.5% relative humidity, open), and illumination (open, illuminated under conditions of 5000 +/- 500 lux (visible light) and 90 w/cm² (ultraviolet) for 10 days, where the total illuminance received was not less than 1.2×10⁶ Lux-hr, and the near-ultraviolet energy was not less than 200 w·hr/m²; samples of a light-shielding control group were placed at the same time and wrapped in aluminum foil). Each sample was placed in an amount of 1.5 g. After the completion of sample placement, all stability samples were subjected to XRPD testing, and the results are shown in Table 9.

**Table 9. Solid stability test results of crystal form A of the compound of formula (I)**

| Test condition | Sampling condition | Crystal form |
|---|---|---|
| - | 0 day | Crystal form A |
| High temperature (60°C, open) | 30 days | Crystal form A |
| High humidity (25°C, 92.5% relative humidity, open) | 30 days | Crystal form A |
| Light-shielding control group | Placed simultaneously with visible light + ultraviolet light, and wrapped in aluminum foil | Crystal form A |
| Visible light + ultraviolet light (ICH conditions, open) | Total illuminance of visible light no less than 1.2 × 10⁶ Lux·hr; Total illuminance of ultraviolet light no less than 200 w·hr/m² | Crystal form A |

Conclusion: Under all the stability conditions (high temperature, high humidity, and illumination), the crystal form A of the compound of formula (I) did not undergo a significant change, indicating that it has good chemical stability.

### Biological assay data

### Experimental example 1: Proliferative inhibitory effect of crystal form A of the compound of formula (I) on GP2D cell line

### I. Experimental materials

**Table 10. Cells and culture medium preparation**

| Cell name | Cell type | Cell source | Catalog No. | Culture medium | Culture conditions |
|---|---|---|---|---|---|
| GP2D | Human colon cancer cells | ECACC | 95090714 | DMEM, containing 10% fetal bovine serum, 1% penicillin/streptomycin | 37°C 5% CO₂ |

**Table 11. Main reagents**

| Reagent | Brand | Brand catalog No. | Lot No. | Storage conditions | Shelf life |
|---|---|---|---|---|---|
| CellTiter-Glo ^{®} 3D Cell Viability assay | Promega | G9683 | 0000575083 | -20°C | 2026.02 |
| 0.25% Trypsin | Gibco | 25200-072 | 2537168 | -20°C | 2025.02 |
| DMSO | Sigma | D2650-100ML | RNBL1802 | Room | NA |
| Penicillin/streptomycin | HyClone | SV30010 | 1220018 | 4°C | 2024.08 |
| Fetal bovine serum, FBS | ExCell Bio | FSP500 | 12B244 | -20°C | 2027.08 |
| DMEM | Gibco | 11965-092 | 2509030 | 4°C | 2023.12 |

### II. Experimental method

Cell culture medium for experiment: Fetal bovine serum at a final concentration of 10% and a penicillin/streptomycin solution at a final concentration of 1% were added into a DMEM cell culture medium, and the mixture was stored at 4°C for future use.

GP2D cells that had reached 80% cell confluence were digested with trypsin, centrifuged, resuspended, and counted. A cell suspension was prepared using the culture medium. The cell suspension was added into a 96-well plate at an amount of 180 µL/well (1000 cells/well). The plate was placed in a cell incubator containing 5% CO₂ and cultured at 37°C overnight.

The test compound was diluted with a DMSO solution to 200 µM, and then further diluted with the complete culture medium to 10 µM. Taking 10 µM as an initial concentration, a 5-fold serial dilution was performed to obtain 9 concentrations. After the cells were cultured overnight, the diluted mixture solutions were respectively transferred to the corresponding cell plates. The final concentration of the compound took 1 µM as an initial concentration, and a 5-fold serial dilution was performed to obtain 9 concentrations. The mixtures were mixed well, and placed in a cell incubator containing 5% CO₂ and cultured at 37°C for 5 days.

After the compound was incubated for the above-mentioned time, the 96-well cell culture plate was taken out. After 50 µL of the supernatant was discarded, 80 µL of 3D-CellTiter Glo was added thereto, and the mixture was incubated with shaking at room temperature for 30 minutes. The plate was read on an Envision plate reader using the *US LUM-US LUM 96 (cps) program.

The measured signal values of each group were normalized by subtracting the signal value of a cell-free control well. Data were calculated according to the following formula for the cell viability after the compound treatment: % Inhibition rate = (1 - (RFU compound - AVER(RFU negative control)) / (AVER(RFU positive control) - AVER(RFU negative control))) x 100%. Positive control: cells treated with 0.5% DMSO; negative control: cell-free wells containing only the complete culture medium. Prism 8.3.0 was used for plotting and calculating the IC₅₀ value of the compound. The calculation formula for the IC₅₀ value is log(inhibitor) vs. response (three parameters): Y = Bottom + (Top - Bottom) / (1 + 10^(X - LogIC₅₀)).

### III. Experimental results

The IC₅₀ values of the compound for inhibiting cell proliferation are shown in the table below.

**Table 12. Inhibitory effect of the compound on proliferation of GP2D cells**

| Compound | GP2D IC₅₀ (nM) |
|---|---|
| Crystal form A of the compound of formula (I) | 1.25 |

Experimental conclusion: The crystal form A of the compound of formula (I) exhibits a significant proliferative inhibitory effect on GP2D cells with a KRAS G12D mutation.

### Experimental example 2: Proliferative inhibitory effect of the compound of formula (I) on PANC04.03 cell line

### I. Experimental objective:

The objective of this experiment is to verify the proliferative inhibitory effect of the compounds of the present disclosure on PANC04.03 human pancreatic cancer cells with a KRAS G12D mutation.

### II. Experimental materials:

The cell line PANC04.03 and the RPMI-1640 culture medium were purchased from GIBCO, FBS was purchased from Hyclone, and human insulin was purchased from Yeasen. 96-Well plates were purchased from Ultra Low Cluster, the CellTiter-Glo (registered trademark) 3D Cell Viability Assay reagent was purchased from Promega, and a 2104 EnVision plate reader was purchased from PerkinElmer.

### III. Experimental method:

PANC04.03 cells were cultured under culture conditions of RPMI-1640 + 15% FBS + 5 ug/mL human insulin in an incubator at 37°C and 5% CO₂. Passaging was performed periodically, and cells in a logarithmic growth phase were collected for plating. The PANC04.03 cells were seeded into a 96-well U-bottom cell culture plate at an amount of 135 L of a cell suspension per well, wherein the cell suspension contained 2000 PANC04.03 cells. The culture plate was cultured overnight in an incubator at 37°C, 5% CO₂, and 100% relative humidity. The compound to be tested was subjected to a 5-fold serial dilution using a multichannel pipette to obtain an 8^{th} concentration, that is, from 200 M down to 2.56 nM, and a duplicate-well experiment was set up. A culture medium (78 µL) was added to an intermediate plate, and then the gradient-diluted compounds were transferred to the intermediate plate at an amount of 2 µL per well according to the corresponding positions. After being mixed well, the mixtures were transferred to the cell plate at an amount of 20 µL per well. The concentration range of the compound transferred to the cell plate was from 1 M to 0.0128 nM. The cell plate was placed and cultured in a CO₂ incubator for 7 days. After the incubation of the cell plate to which the compound was added was completed, a cell viability chemiluminescence detection reagent was added to the cell plate at an amount of 100 µL per well, and the mixture was incubated at room temperature for 10 minutes to stabilize the luminescence signal. A multilabel analyzer was used for reading.

### IV. Data analysis:

The raw data were converted into an inhibition rate using the equation: (Sample - Min) / (Max - Min) x 100%. The IC₅₀ value can be obtained through a curve fitting with four parameters (obtained by using the "log(inhibitor) vs. response -- Variable slope" mode in GraphPad Prism).

### V. Experimental results:

The results are shown in Table 13.

**Table 13. Anti-proliferative IC₅₀ values of the compound against PANC04.03 cells**

| **Compound** | **PANC04.03 IC₅₀ (nM)** |
|---|---|
| Compound of formula (I) | 34.17 |

**Experimental conclusion:** The compound of formula (I) has a significant anti-proliferative activity against PANC04.03 cells with a KRAS G12D mutation.

### Experimental example 3: In vivo pharmacokinetic experiment

### I. Experimental objective:

The objective of this experiment is to investigate the pharmacokinetic characteristics of the compounds of the present disclosure in CD-1 mice via oral administration and intravenous injection.

### II. Experimental method:

The test compound was mixed with 10% dimethyl sulfoxide + 90% (10% hydroxypropyl-β-cyclodextrin (HP-β-CD) aqueous solution), and the mixture was vortexed and ultrasonicated to respectively prepare clear solutions of 0.6, 3.0, and 10.0 mg/mL. Male CD-1 mice aged 7 to 10 weeks were selected. The candidate compound solution was administered via intravenous injection (i.v.) at a dose of 3 mg/kg (administration concentration: 0.6 mg/mL). The candidate compound solution was administered via oral gavage (p.o.) at a dose of 30 mg/kg (administration concentration: 3.0 mg/mL) or 100 mg/kg (administration concentration: 10.0 mg/mL). Whole blood was collected at specific time points to prepare plasma. The drug concentration was analyzed by an LC-MS/MS method, and the pharmacokinetic parameters were calculated using Phoenix WinNonlin software (Pharsight, USA).

### III. Experimental results:

The results are shown in Table 14.

**Table 14. PK properties of the compound of formula (I) in CD-1 mice**

| Compound of formula (I) | | |
|---|---|---|
| i.v. 3 mpk | T_{1/2} (h) | 9.30 |
| | Vd (L/kg) | 41.1 |
| | Cl (mL/kg/min) | 50.8 |
| | AUC₀₋ₗₐₛₜ (nM.h) | 1556 |
| | AUC_{0-∞} (nM.h) | 1660 |
| p.o. 30 mpk | T_{1/2} (h) | 5.41 |
| | Tₘₐₓ (h) | 0.583 |
| | Cₘₐₓ (nmol/L) | 439 |
| | AUC₀₋ₗₐₛₜ (nM.h) | 1154 |
| | AUC_{0-∞} (nM.h) | 1183 |
| p.o. 100 mpk | T_{1/2} (h) | 4.24 |
| | Tₘₐₓ (h) | 1.25 |
| | Cₘₐₓ (nmol/L) | 3114 |
| | AUC₀₋ₗₐₛₜ (nM.h) | 13149 |
| | AUC_{0-∞} (nM.h) | 13306 |
| | F (%) | 24.0 |

**Experimental conclusion:** The compound of formula (I) has good pharmacokinetic characteristics in mice.

## Claims

1. A crystal form A of a compound of formula (I), wherein the crystal form A has an X-ray powder diffraction pattern using Cu Kα radiation comprising characteristic diffraction peaks at the following 2θ angles: 8.09°±0.2°, 21.34°±0.2°, 24.74°±0.2°, and 25.26°±0.2°,

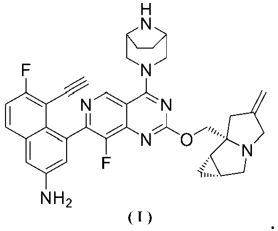

2. The crystal form A according to claim 1, wherein the X-ray powder diffraction pattern thereof using Cu Kα radiation comprises characteristic diffraction peaks at the following 2θ angles: 8.09°±0.2°, 19.18°±0.2°, 19.49°±0.2°, 21.34°±0.2°, 24.74°±0.2°, and 25.26°±0.2°.

3. The crystal form A according to claim 2, wherein the X-ray powder diffraction pattern thereof using Cu Kα radiation comprises characteristic diffraction peaks at the following 2θ angles: 8.09°±0.2°, 9.46°±0.2°, 13.95°±0.2°, 19.18°±0.2°, 19.49°±0.2°, 21.34°±0.2°, 24.74°±0.2°, and 25.26°±0.2°.

4. The crystal form A according to claim 3, wherein the X-ray powder diffraction pattern thereof using Cu Kα radiation comprises characteristic diffraction peaks at the following 2θ angles: 8.09°±0.2°, 9.46°±0.2°, 13.95°±0.2°, 15.28°±0.2°, 17.12°±0.2°, 19.18°±0.2°, 19.49°±0.2°, 21.34°±0.2°, 24.74°±0.2°, and 25.26°±0.2°.

5. The crystal form A according to claim 4, wherein the X-ray powder diffraction pattern thereof using Cu Kα radiation comprises characteristic diffraction peaks at the following 2θ angles: 8.09°±0.2°, 9.46°±0.2°, 13.95°±0.2°, 15.28°±0.2°, 17.12°±0.2°, 19.18°±0.2°, 19.49°±0.2°, 20.82°±0.2°, 21.34°±0.2°, 24.74°±0.2°, 25.26°±0.2°, and 28.32°±0.2°.

6. The crystal form A according to claim 5, the X-ray powder diffraction pattern thereof using Cu Kα radiation comprises characteristic diffraction peaks at the following 2θ angles: 8.09°±0.2°, 9.46°±0.2°, 12.10°±0.2°, 13.95°±0.2°, 15.28°±0.2°, 16.39°±0.2°, 17.12°±0.2°, 19.18°±0.2°, 19.49°±0.2°, 20.82°±0.2°, 21.34°±0.2°, 22.22°±0.2°, 24.74°±0.2°, 25.26°±0.2°, 27.99°±0.2°, and 28.32°±0.2°.

7. The crystal form A according to claim 6, wherein the X-ray powder diffraction pattern thereof using Cu Kα radiation comprises characteristic diffraction peaks at the following 2θ angles: 8.09°±0.2°, 9.46°±0.2°, 12.10°±0.2°, 12.78°±0.2°, 13.06°±0.2°, 13.95°±0.2°, 15.28°±0.2°, 16.39°±0.2°, 17.12°±0.2°, 18.56°±0.2°, 18.86°±0.2°, 19.18°±0.2°, 19.49°±0.2°, 19.84°±0.2°, 20.82°±0.2°, 21.34°±0.2°, 22.22±0.2°, 22.96°±0.2°, 23.58°±0.2°, 24.11°±0.2°, 24.74°±0.2°, 25.26°±0.2°, 27.99°±0.2°, 28.32°±0.2°, 29.00°±0.2°, and 30.21°±0.2°.

8. The crystal form A according to claim 7, wherein the X-ray powder diffraction pattern thereof using Cu Kα radiation comprises characteristic diffraction peaks at the following 2θ angles: 7.29°±0.2°, 8.09°±0.2°, 9.46°±0.2°, 9.75°±0.2°, 11.06°±0.2°, 12.10°±0.2°, 12.78°±0.2°, 13.06°±0.2°, 13.95°±0.2°, 14.87°±0.2°, 15.28°±0.2°, 16.39°±0.2°, 17.12°±0.2°, 17.54°±0.2°, 18.56°±0.2°, 18.86°±0.2°, 19.18°±0.2°, 19.49°±0.2°, 19.84°±0.2°, 20.82°±0.2°, 21.34°±0.2°, 22.22°±0.2°, 22.96°±0.2°, 23.58°±0.2°, 24.11°±0.2°, 24.74°±0.2°, 25.26°±0.2°, 25.73°±0.2°, 26.11°±0.2°, 26.50°±0.2°, 26.64°±0.2°, 27.54°±0.2°, 27.99°±0.2°, 28.32°±0.2°, 28.78°±0.2°, 29.00°±0.2°, 30.21°±0.2°, 30.50°±0.2°, 31.03°±0.2°, 31.99°±0.2°, 33.33°±0.2°, 33.69°±0.2°, 34.01°±0.2°, 34.39°±0.2°, 34.81°±0.2°, 35.48°±0.2°, 35.82°±0.2°, 36.37°±0.2°, 36.63°±0.2°, 37.34°±0.2°, 37.78°±0.2°, 38.39°±0.2°, 38.71°±0.2°, 39.14°±0.2°, and 39.57°±0.2°.

9. The crystal form A according to claim 8, wherein the crystal form A has an XRPD pattern substantially as shown in FIG. 1.

10. The crystal form A according to any one of claims 1 to 9, wherein the crystal form A has a differential scanning calorimetry (DSC) curve comprising an exothermic peak with an onset at 199.55±3.00°C.

11. The crystal form A according to any one of claims 1 to 9, wherein the crystal form A has a DSC pattern substantially as shown in FIG. 2.

12. The crystal form A according to any one of claims 1 to 9, wherein the crystal form A has a thermogravimetric analysis (TGA) curve showing a weight loss of 4.502% at 220.00±3.00°C.

13. The crystal form A according to claim 12, wherein the crystal form A has a TGA pattern substantially as shown in FIG. 3.

14. A pharmaceutical composition comprising the crystal form A of the compound of formula (I) according to any one of claims 1 to 13; optionally, the pharmaceutical composition further comprises a pharmaceutically acceptable excipient.

15. Use of the crystal form A of the compound of formula (I) according to any one of claims 1 to 13 or the pharmaceutical composition according to claim 14 in the manufacture of a medicament for treating a KRAS G12D-mutated solid tumor;
preferably, the KRAS G12D-mutated solid tumor is selected from colon cancer and pancreatic cancer.
